# FASCICULE DE BREVET EUROPEEN

(11) **EP 1 294 297 B1**
(45) Date de publication et mention de la délivrance du brevet: **11.08.2010**
(21) Numéro de dépôt: 01949595.1
(22) Date de dépôt: 29.06.2001
(51) Int. Cl.: A61B 17/70

(54) **DISPOSITIF DE LIAISON INTERVERTEBRAL**
ZWISCHENWIRBEL-VERBINDUNGSVORRRICHTUNG
INTERVERTEBRAL LINKING DEVICE

(30) Priorité: 30.06.2000 FR 0008522; 01.08.2000 FR 0010155
(43) Date de publication de la demande: 26.03.2003
(73) Titulaire: Warsaw Orthopedic, Inc., Warsaw, IN 46581 (US)
(72) Inventeur: Graf, Henry, F-69006 Lyon (FR)
(74) Mandataire: Neyret, Daniel Jean Marie
(86) Numéro de dépôt international: PCT/FR2001/002098
(87) Numéro de publication internationale: WO 2002/000124

(56) Documents cités:
- WO-A-00/15125
- US-A- 5 672 176
- US-A- 5 683 392
- US-A- 5 690 630
- US-A- 5 733 285

## Description

La présente invention concerne un dispositif de liaison intervertébral.

On connaît un tel dispositif qui comprend au moins deux vis pédiculaires, dont chacune possède une première extrémité solidarisée à un corps vertébral correspondant, une portion intermédiaire renflée, ainsi qu'une seconde extrémité filetée. Des organes auxiliaires, pourvus d'une arche de fixation d'une tige s'étendant entre les vertèbres, sont disposés sur chacune des portions renflées précitées. Un boulon, coopérant avec l'extrémité filetée de chaque vis, permet l'immobilisation de chaque organe auxiliaire, une fois ce dernier mis en place de façon appropriée.

Ce dispositif connu présente cependant certains inconvénients, en ce sens qu'il implique un procédé de montage relativement délicat. Par ailleurs, une fois implanté, il n'offre aucun degré de liberté entre les différents éléments qui le constituent. Ainsi, lorsque des efforts s'exercent au niveau des corps vertébraux, cette absence de degré de liberté induit une transmission de ces efforts sur l'ensemble du dispositif, de sorte que ce dernier a tendance à se désolidariser des vertèbres qu'il relie et induit par ailleurs des dysfonctionnements au niveau de l'ensemble de la chaîne vertébrale. WO 00/15125 montre un dispositif avec un élément intermédiaire déformable 36.

Afin de pallier ces différents inconvénients, la présente invention se propose de réaliser un dispositif dont la structure est simple, dont le montage est aisé et qui est implanté de façon fiable dans les vertèbres qu'il relie.

A cet effet, elle a pour objet un dispositif de liaison intervertébral tel que défini dans la revendication 1.

L'invention va être décrite ci-dessous, en référence aux dessins annexés, donnés uniquement à titre d'exemples non limitatifs et dans lesquels :
- la figure 1 est une vue en coupe longitudinale, illustrant les différents éléments constitutifs d'un dispositif de liaison intervertébral conforme à un premier mode de réalisation de l'invention;
- les figures 2, 2A et 2B sont des vues analogues à la figure 1, illustrant trois étapes du montage d'un élément intermédiaire du dispositif de la figure 1, dans le volume intérieur d'un élément mobile de ce dispositif ;
- les figures 3 et 3A sont des vues analogues figure 1, illustrant l'introduction d'un élément fixe du dispositif de la figure 1, dans le volume intérieur de son élément intermédiaire ;
- les figures 4 à 5 sont des vues analogues à la figure 3A, illustrant des variantes de réalisation du dispositif conforme à l'invention ;
- la figure 6 montre une variante non couverte par l'invention
- les figures 7 et 8 sont des vues en coupe respectivement longitudinale et transversale, d'un dispositif conforme à un mode supplémentaire de réalisation de l'invention,
- les figures 9 et 10 sont des vues en coupe longitudinale, illustrant deux variantes supplémentaires de réalisation de l'invention ;
- la figure 11 est une vue en perspective, illustrant les différents éléments d'un dispositif conforme à une variante supplémentaire de l'invention ;
- la figure 12 est une vue en coupe longitudinale du dispositif de la figure 11, une fois monté ; et
- les figures 13 à 15 sont des vues en perspective, analogues à la figure 11, illustrant trois variantes supplémentaires de réalisation de l'invention.

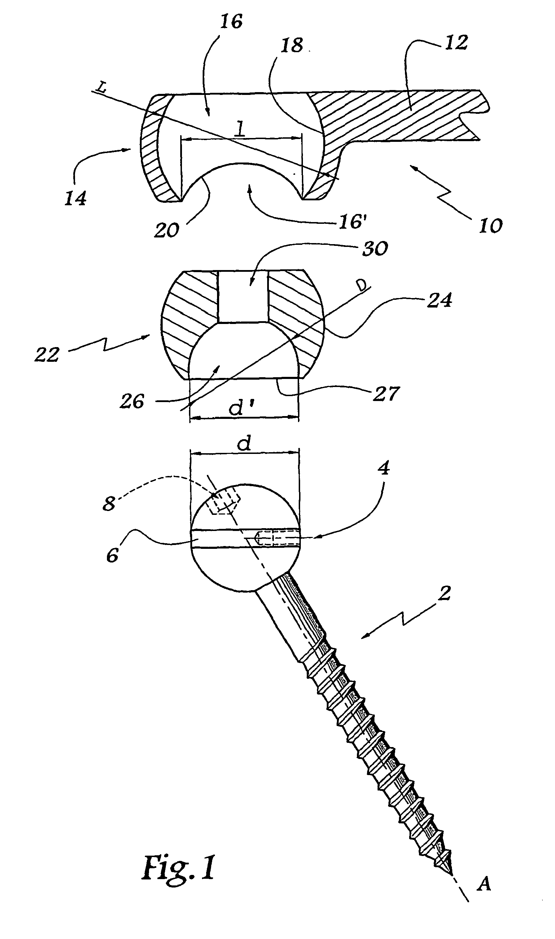

La figure 1 illustre un premier mode de réalisation du dispositif de liaison conforme à l'invention, qui comprend une vis pédiculaire 2, destinée à être solidarisée dans un corps vertébral non représenté. Cette vis pédiculaire, qui constitue ainsi un élément fixe, est pourvue d'une tête sphérique 4 comportant un méplat équatorial 6 s'étendant de façon inclinée, en ce sens qu'il n'est pas perpendiculaire à l'axe principal A de la vis 2. La tête 4 est en outre creusée d'un trou borgne 8, destiné à la réception d'un organe de manoeuvre, notamment de l'extrémité d'un tournevis non représenté.

Le dispositif représenté à la figure 1 comprend également un élément mobile, illustré de façon partielle, qui est désigné dans son ensemble par la référence 10. Cet élément mobile possède un corps 12, qui s'étend entre les deux vertèbres que relie le dispositif de l'invention et qui est terminé par deux extrémités creuses, dont une seule 14 est représentée.

Chaque extrémité définit un logement 16, formant un volume intérieur de l'élément 10, qui est bordé par des parois 18 formant un tronçon de sphère. Ces parois possèdent une échancrure 20, permettant d'élargir localement la section de passage du logement 16, de manière à permettre l'introduction d'un élément intermédiaire, comme cela sera décrit dans ce qui suit. Par ailleurs, la dimension transversale 1 du débouché 16' du logement 16 est inférieure au diamètre L de ce logement.

Le dispositif de la figure 1 comprend enfin un élément intermédiaire 22, dont la surface extérieure 24 forme une portion de sphère, dont le diamètre est identique à celui L du logement 16. L'élément intermédiaire 22 est creusé d'une ouverture traversante, qui définit un logement 26, formant un volume intérieur bordé par des parois sphériques tronquées, dont le diamètre D est identique à celui de la tête 4. Le logement 26 communique avec une chambre 30 permettant le passage de l'organe de manoeuvre précité, en direction du trou borgne 8.

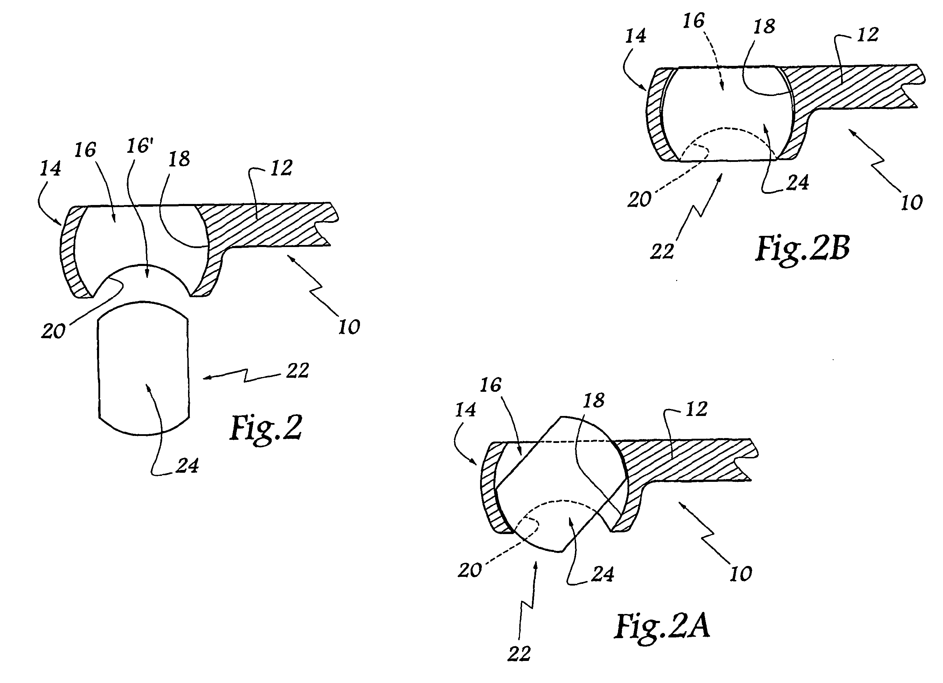

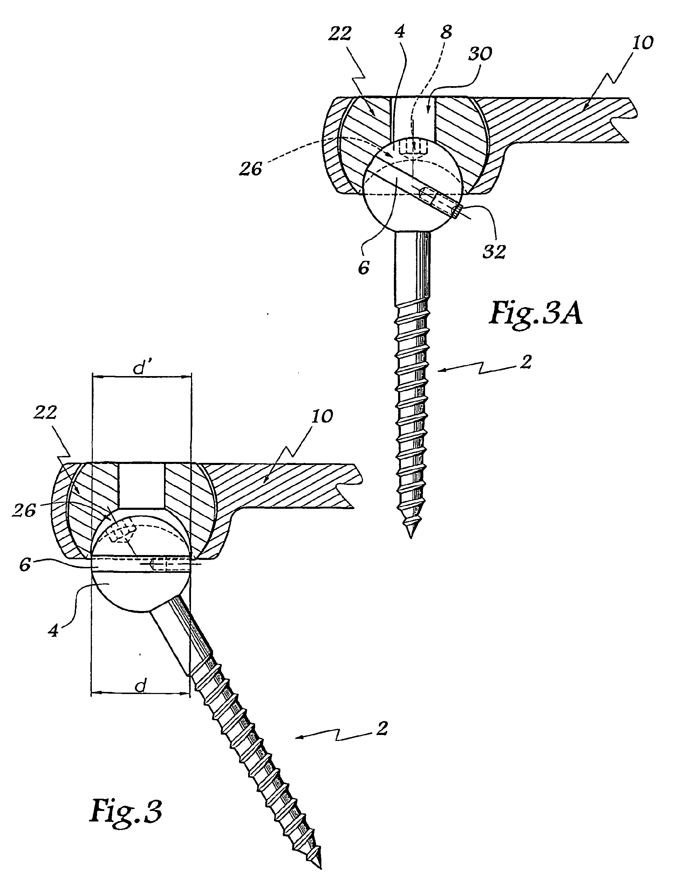

Le montage du dispositif illustré à la figure 1, va être décrit en référence aux figures 2 et 3.

Il s'agit, dans un premier temps, d'introduire l'élément intermédiaire 22 dans le volume intérieur 16 de l'élément mobile 10. A cet effet, on dispose cet élément intermédiaire 22, de sorte que sa surface extérieure sphérique 24 se trouve au voisinage du débouché 16'. Puis, on rapproche axialement l'un de l'autre l'élément intermédiaire 22 et l'élément mobile 10. L'élément intermédiaire et l'élément mobile ne sont pas liés en rotation ni en translation, dans cette position d'introduction.

On fait ensuite pivoter l'élément intermédiaire 22 autour de son axe, de manière que sa surface extérieure 24 s'étende au voisinage des parois intérieures 18, comme le montre la figure 2C. Une fois ces opérations réalisées, l'élément intermédiaire 22 ne possède aucun degré de liberté en translation par rapport à l'élément mobile, dans cette position d'utilisation. En effet, la dimension transversale 1 du débouché 16' est inférieure au diamètre extérieur de l'élément intermédiaire 22. En revanche, ce dernier possèd trois degrés de liberté en rotation par rapport à l'élémen mobile 10.

Puis, comme le montre la figure 3, il s'agit d'introduir la tête sphérique 4 de la vis pédiculaire 2 dans le logement 26 de l'élément intermédiaire 22. A cet effet, on incline tout d'abord cette vis 2, de sorte que le méplat 6 s'étende horizontalement sur cette figure 3, à savoir de manière perpendiculaire à l'axe principal de l'élément intermédiaire 22.

On rapproche alors cet élément intermédiaire 22 de la vis 2, selon une translation parallèle à l'axe principal de cet élément intermédiaire 22. Etant donné que la dimension transversale d du méplat est égale, ou légèrement inférieure, à la dimension transversale d' du débouché 27 du logement 26, ceci permet une libre introduction de la tête 4 dans ce logement 26, illustrée à la figure 3.

Le pourtour du débouché 27 est sensiblement rigide, c'est-à-dire non déformable. A cet effet, l'élément intermédiaire 22 peut être réalisé entièrement en un matériau rigide, notamment métallique. En variante, cet élément intermédiaire peut être réalisé en un matériau déformable, tel que du polyéthylène, une bague rigide étant alors rapportée au voisinage de ce débouché.

Ensuite, on fait pivoter la tête 4 à l'intérieur du logement, de manière que le méplat 6 soit à nouveau incliné, c'est-à-dire qu'il ne se trouve plus en regard du débouché 27 précité. La tête 4 est alors libre de pivoter par rapport à ce logement 26, mais ne possède aucun degré de liberté en translation par rapport à l'élément intermédiaire 22, étant donné que le diamètre D de la tête 4 est supérieur à la dimension transversale du débouché 27 du logement 26.

Une fois le dispositif mis dans la configuration illustrée à la figure 3A, il s'agit de fixer la vis pédiculaire 2 dans un corps vertébral correspondant, au moyen d'un organe de manoeuvre coopérant avec le trou borgne 8 de cette vis pédiculaire 2.

A titre de variante de montage, il est possible de tout d'abord chaque vis pédiculaire dans un corps vertébral correspondant. Puis, on introduit chaque élément intermédiaire 22 dans le volume intérieur 16 de l'élément mobile 10, comme cela est expliqué en référence à la figure 2.

On rapproche alors mutuellement l'élément fixe et l'élément mobile, et on fait basculer l'élément intermédiaire 22 au sein de son logement 6. On rapproche ensuite l'élément intermédiaire 22 ainsi basculé, par rapport à la vis pédiculaire 2, de manière que le méplat 6 coopère avec le pourtour du débouché 27, comme cela est illustré à la figure 3.

Une fois le dispositif de l'invention placé dans la configuration de cette figure 3A, on peut rapporter, sur le méplat 6, un moyen de butée avantageusement amovible, telle une vis 32. Cette dernière, en limitant le pivotement de la tête 4 par rapport à l'élément intermédiaire 22, empêche cette tête de recouvrer sa position à la figure 3, ce qui évite toute désolidarisation entre l'élément intermédiaire 22 et la vis pédiculaire 2.

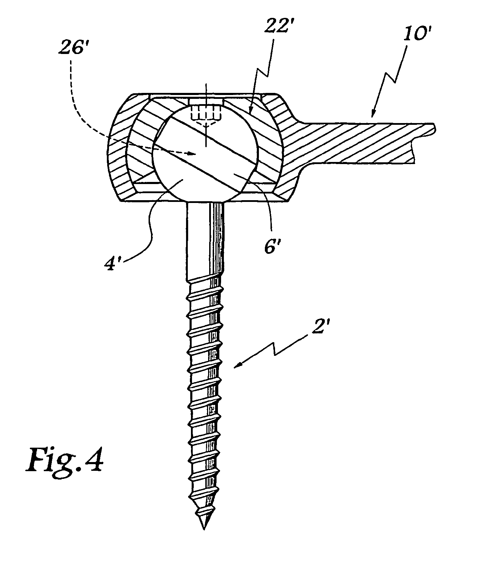

La figure 4 illustre une variante de réalisation de l'invention, dans laquelle la vis pédiculaire 2' est pourvue d'une tête sphérique 4' elle-même pourvue d'un méplat équatorial incliné 6'. Cette tête 4' est introduite, de façon analogue à ce qui a été décrit précédemment, dans un élément intermédiaire 22' pourvu d'un volume intérieur 26'.

Il est à noter que, contrairement à l'exemple décrit en référence aux figures 1 à 3, la tête sphérique 4' et le volume intérieur 26' sont concentriques. Par ailleurs, l'élément intermédiaire 22' est reçu dans un volume intérieur 16' d'un élément mobile 10', de façon analogue à ce qui a été décrit ci-dessus.

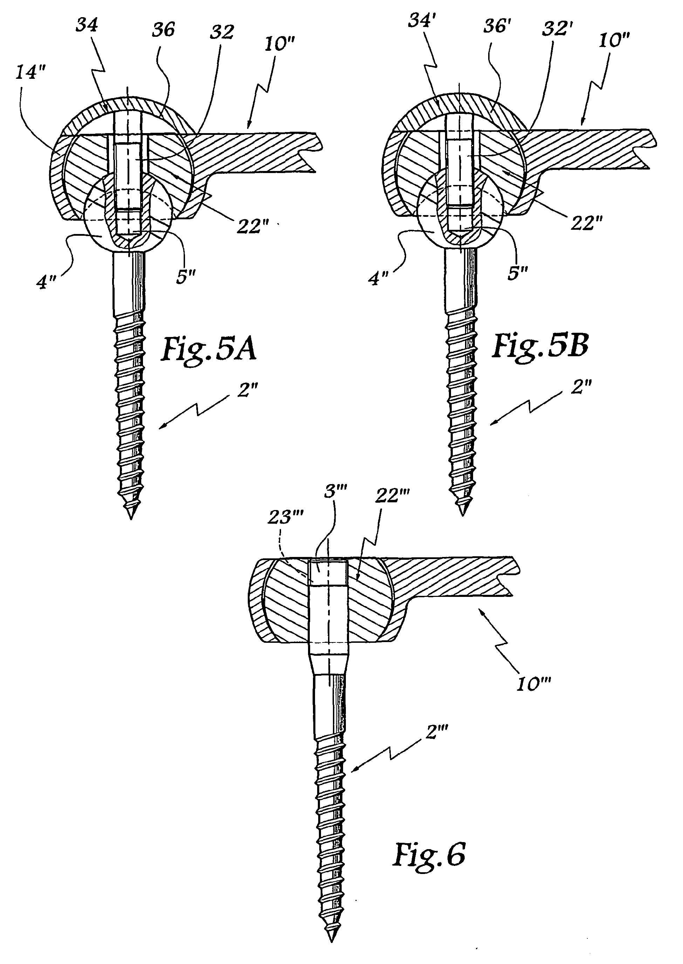

Les figures 5 et 5A illustrent des variantes de réalisation dans lesquelles il est prévu des moyens de fixation amovibles, permettant de solidariser la vis pédiculaire 2", soit avec l'élément mobile 10", soit avec l'élément intermédiaire 22". A cet effet, la tête 4" de la vis pédiculaire est pourvue d'un taraudage 5", coopérant avec une tige filetée 32 d'un élément de fixation 34. Ce dernier comprend également un dôme 36 en forme de champignon, prenant appui les parois de l'extrémité 14" de l'élément mobile 10".

Dans le mode de réalisation de la figure 5A, le dôme 36' de l'élément de fixation 34' prend appui également sur l'extrémité de l'élément intermédiaire 22" , alors que la tige filetée 32' est solidarisée avec la tête sphérique 4" de la vis pédiculaire.

La figure 6 illustre une variante non couverte par l'invention, dans laquelle l'élément intermédiaire 22"' est creusé d'un taraudage 23"'. Ce dernier coopère avec une extrémité filetée 3"' de la vis pédiculaire 2"', qui est dépourvue de tête sphérique. L'élément intermédiaire 22"' est par ailleurs reçu, à rotule, dans l'élément mobile 10"', comme dans les exemples précédents.

A titre de variante on peut remplacer la vis pédiculaire décrite ci-dessus par une tige s'étendant depuis une plaque sacrée, à savoir destinée à être vissée sur le sacrum.

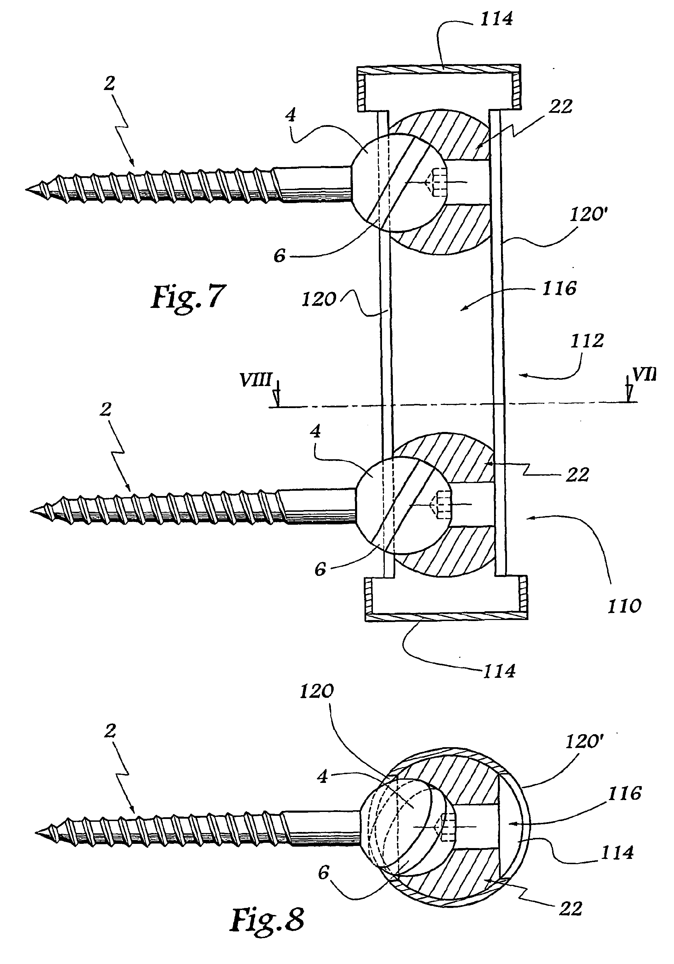

Les figures 7 et 8 illustrent un autre mode de réalisation de l'invention, dans lequel il est fait appel à au moins deux vis pédiculaire 2, ainsi qu'à au moins deux éléments intermédiaires 22, identiques à ceux décrits en référence aux figures 1 à 3.

L'élément mobile 110 comprend un corps tubulaire 112, qui est terminé par deux extrémités fermées 114 et se trouve creusé d'une première échancrure longitudinale 120, dont le pourtour est sensiblement rigide, permettant d'introduire chaque élément intermédiaire 22 dans le volume intérieur 116 de l'élément mobile 110.

Cette introduction est réalisée de façon analogue à celle décrite en référence aux figures 2, 2A et 2B, au moyen d'un pivotement de l'élément intermédiaire d'un quart de tour. Par ailleurs, il est prévu une seconde échancrure longitudinale 120', diamétralement opposée à celle 120, permettant le passage d'un organe de manoeuvre, en vue de la fixation de chaque vis 2 dans les vertèbres correspondantes.

Ce mode de réalisation confère trois degrés de liberté en rotation à l'élément intermédiaire 22 par rapport à l'élément mobile 110, et permet également un coulissement axial de cet élément intermédiaire, le long du corps cylindri-que 112. En variante, la tête 4 de chaque vis peut être concentrique par rapport à l'élément intermédiaire, comme dans l'exemple de réalisation décrit à la référence à la figure 4.

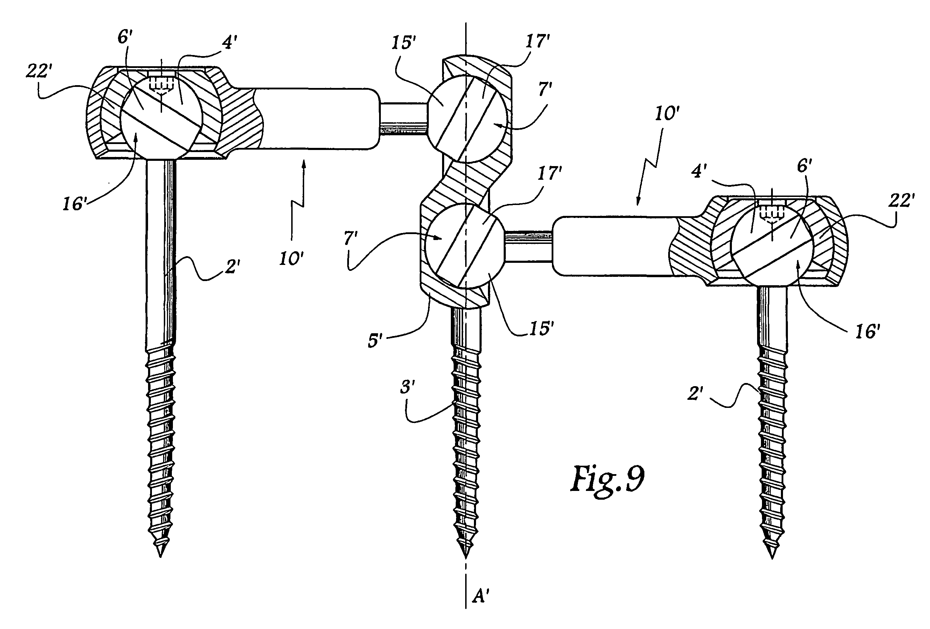

La figure 9 illustre un dispositif permettant de relier trois vertèbres voisines.

Ce dispositif comprend deux vis d'extrémité 2', analogues à celles de la figure 4. Chaque vis comporte une tête 4' pourvue d'un méplat 6', coopérant avec un élément intermédiaire 22'. Ce dernier est reçu dans le volume intérieur 16' d'un élément mobile 10', qui est terminé, à l'opposé de son extrémité recevant l'élément intermédiaire 22', par une tête sphérique 15'.

Par ailleurs, il est prévu une vis supplémentaire 3', placée en position médiane. Elle comprend une tête 5' allongée, dans laquelle sont ménagés deux logements 7', dont les parois sphériques tronquées s'étendent selon un secteur angulaire supérieur à 180°.

Ces deux logements sont ouverts à l'opposé l'un de l'autre, de façon sensiblement perpendiculaire à l'axe principal de cette vis 3'. La dimension transversale du débouché de chaque logement 7' est inférieure au diamètre de ses parois sphériques. La tête sphérique 15' de chaque élément mobile 10', qui est pourvue d'un méplat équatorial incliné 17', coopère avec l'un des logements 7' de la vis médiane 3'.

En vue du montage de la prothèse, il s'agit tout d'abord d'introduire chaque tête sphérique 15' dans le volume intérieur d'un logement 7' correspondant, comme cela a été décrit précédemment pour l'introduction de la tête 4 de la vis 2, dans le volume intérieur de l'élément intermédiaire 22'.

Puis, on introduit chaque élément intermédiaire 22' dans le volume intérieur d'un élément mobile 10'. Enfin, on introduit la tête 4' de chaque vis d'extrémité 2', dans le volume intérieur d'un élément intermédiaire 22' correspondant.

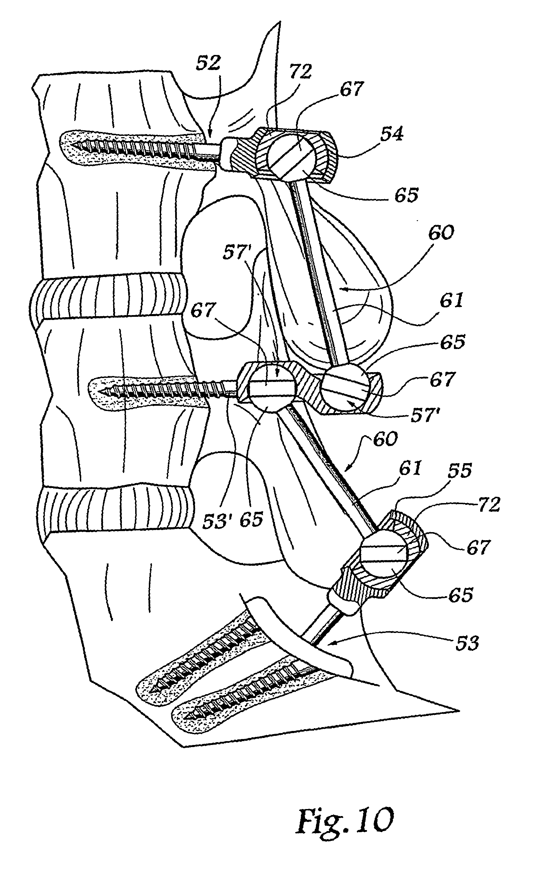

La figure 10 illustre un dispositif permettant de relier deux vertèbres voisines, ainsi que le sacrum.

Il est prévu deux éléments mobiles 60 analogues, dont chacun comprend une tige 61. Cette dernière est terminée, à ses deux extrémités, par une tête sphérique 65 munie de méplats inclinés inclinés 67.

Il est par ailleurs prévu une vis médiane 53' analogue à celle 3' de la figure 9. Chaque tête shérique 65 est reçu dans un logement 57' correspondant, appartenant à cette vis médiane 53'.

Par ailleurs, il est prévu deux éléments fixes d'extrémité, à savoir une vis pédiculaire 52, ainsi qu'une plaque 53, fixée sur le sacrum. A son extrémité opposée à la vis médiane 53', chaque élément mobile 60 est reçu dans un élément intermédiaire 72, analogue à ceux 22, 22', 22" et 22"' décrit ci-dessus.

Par ailleurs, cet élément intermédiaire 72 est reçu dans le volume intérieur d'une tête 54, 55, appartenant respectivement à la vis pédiculaire 52 ou à la plaque sacrée 53.

La liaison mutuelle entre la tête 65, l'élément intermédiaire 72, et la vis 52 ou la plaque 53, est analogue à celle existant entre, par exemple, la tête 4 de la vis 2, l'élément intermédiaire 22 et l'élément mobile 10.

En d'autres termes, dans cette figure 10, la tête 65 se substitue à la tête 4, le volume intérieur de la vis 52 ou de la plaque 53 se substitue au volume intérieur de l'élément mobile 10, alors que l'élément intermédiaire 72 assure l'articulation de l'élément mobile 60 et de la vis 52, tout comme l'élément intermédiaire 22 assure l'articulation de la vis 2 et de l'élément mobile 10.

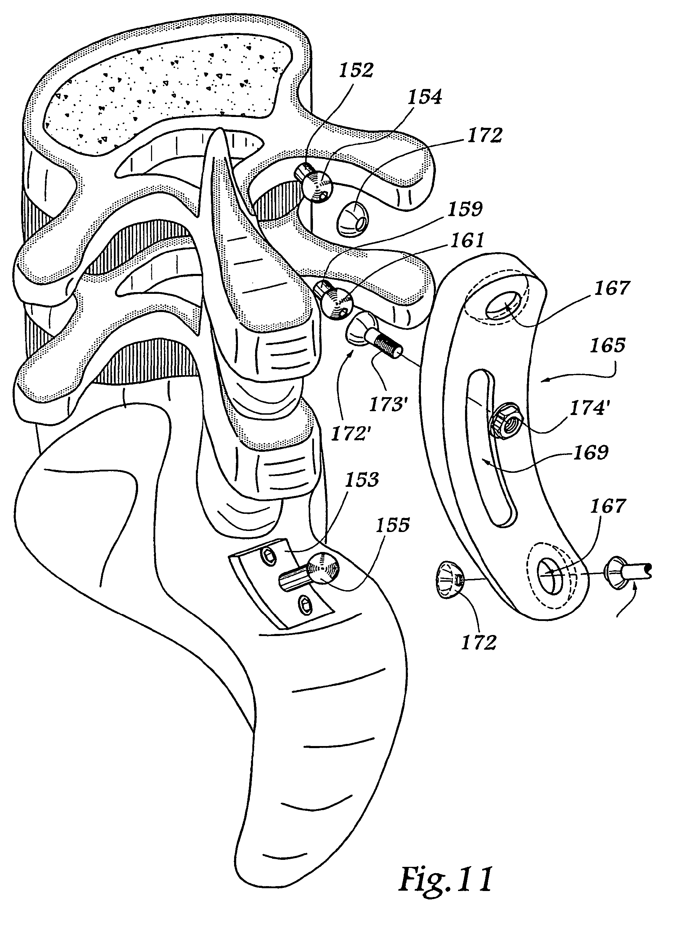

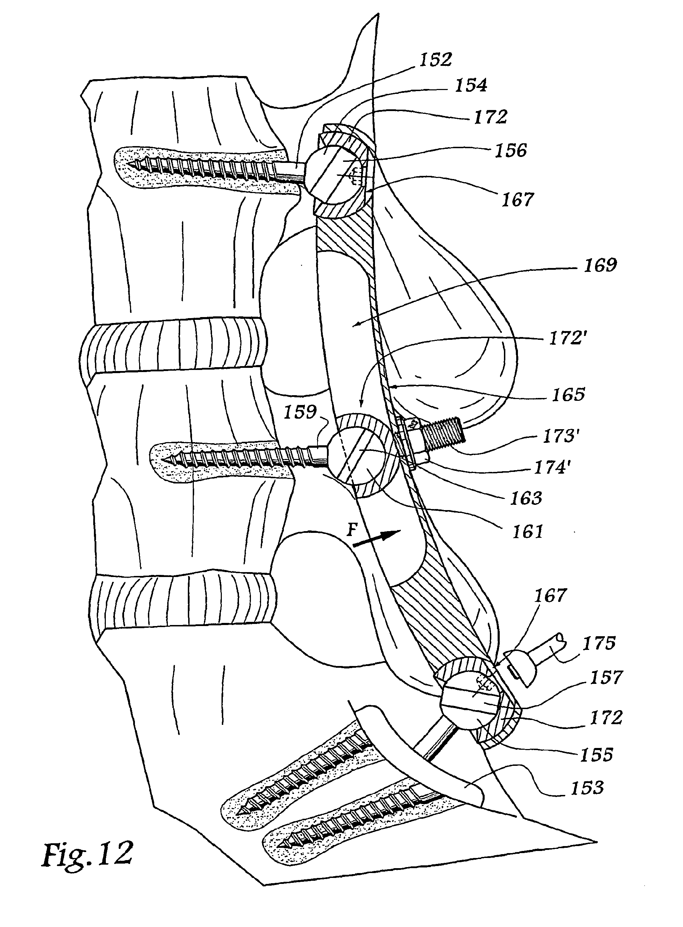

Les figures 11 et 12 illustrent un dispositif permettant de relier mutuellement deux vertèbres lombaires, ainsi que le sacrum.

Ce dispositif comporte une vis pédiculaire supérieure 152, terminée par une tête sphérique 154 pourvue d'un méplat incliné 156. Par ailleurs, il est prévu une plaque sacrée 153, terminée par une tête sphérique 155 pourvue d'un méplat incliné 157. Ce dispositif comporte également une vis médiane 159 terminée par une tête sphérique 161 pourvue d'un méplat équatorial incliné 163.

Il est en outre prévu une plaque allongée 165, dont chaque extrémité est creusée d'une alvéole correspondante 167 Chaque alvéole est analogue au logement 16 représenté figure 1. Cette plaque allongée 165 est par ailleurs creusée d'une lumière oblongue 169.

La tête 154, 155 de la vis d'extrémité 152 ou de la plaque 153 est reçue dans une alvéole 167 correspondante, avec interposition d'un élément intermédiaire 172, analogue notamment à celui 22. La tête 161 de la vis médiane 159 est reçue dans le volume intérieur d'un élément intermédiaire 172', qui diffère de celui 172, en ce sens qu'il est pourvu, à l'opposé de son débouché, d'une tige filetée 173', susceptible de coopérer avec un écrou 174'.

Le montage du dispositif des figures 11 et 12 va maintenant être explicité.

Il s'agit tout d'abord d'introduire chaque élément intermédiaire 172 dans une alvéole 167 correspondante, comme cela a été décrit en référence aux figures 2 à 2B. Puis, on rapproche la plaque allongée 165 de la vis 152 et de la plaque sacrée 153.

On introduit alors les têtes 154, 155 dans le volume intérieur de chaque élément intermédiaire 172. Pour ce faire, il peut être judicieux de faire pivoter ces éléments intermédiaires, sans pour cela déplacer en translation la plaque allongée 165, par rapport à la vis 152 et à la plaque 153. Ceci peut être réalisé par l'intermédiaire d'un palpateur 175, dont l'extrémité traverse l'ouverture de l'alvéole 167 opposée aux vertèbres.

Il est à noter que, avant de rapprocher la plaque allongée 165 des vertèbres, l'élément intermédiaire 172' a été placé sur la tête sphérique 161 de la vis médiane 159. La tige 173' de cet élément intermédiaire traverse alors la lumière 169 de la plaque. Il est ainsi possible, par vissage de l'écrou 174' sur la tige filetée 173', de déplacer, par rapport à la plaque allongée 165, la vis médiane 159 selon la flèche F. Ceci est particulièrement avantageux, dans la mesure où cela permet d'induire un déplacement de la dernière vertèbre lombaire selon cette flèche F, c'est-à-dire de "tirer" cette vertèbre.

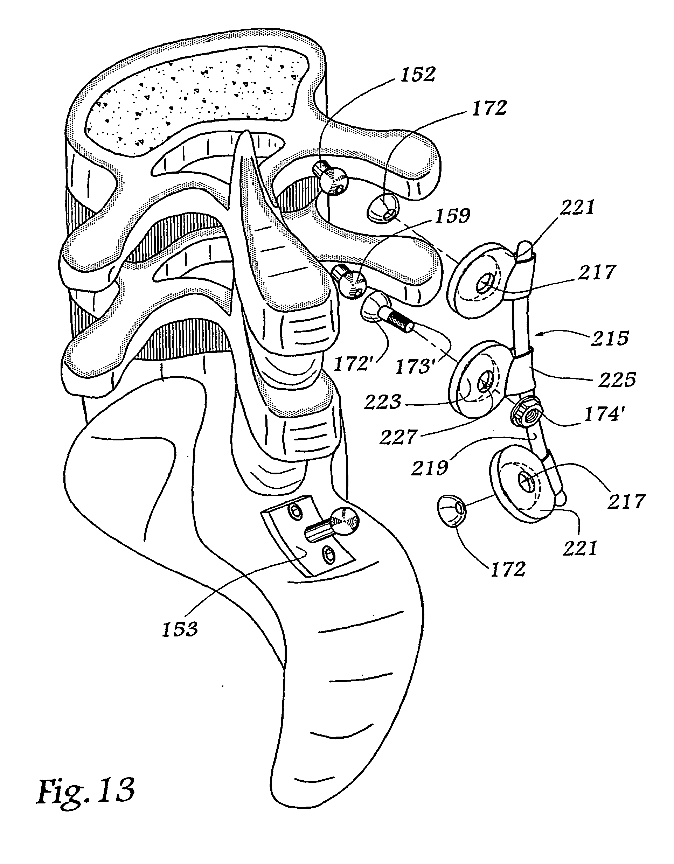

La figure 13 illustre une variante supplémentaire de réalisation de l'invention, dans laquelle la plaque all 165 est remplacée par un organe de liaison 215, qui comporte une tige 219, terminée par deux disques 221, dont chacun est pourvu d'une alvéole 217, analogues à celles 167.

Il est par ailleurs prévu un disque intermédiaire 223, susceptible d'être fixé sur la tige 219 par un collier 225. Ce disque 223 est creusé d'un orifice médian 227.

Chaque disque d'extrémité 221 est susceptible de coopérer, avec interposition d'un élément intermédiaire 172, avec la vis 152 ou la plaque sacrée 153. Par ailleurs, la tige filetée 173' de l'élément intermédiaire 172' est susceptible de traverser l'orifice 227 du disque intermédiaire 223, de façon analogue à la lumière oblongue 169.

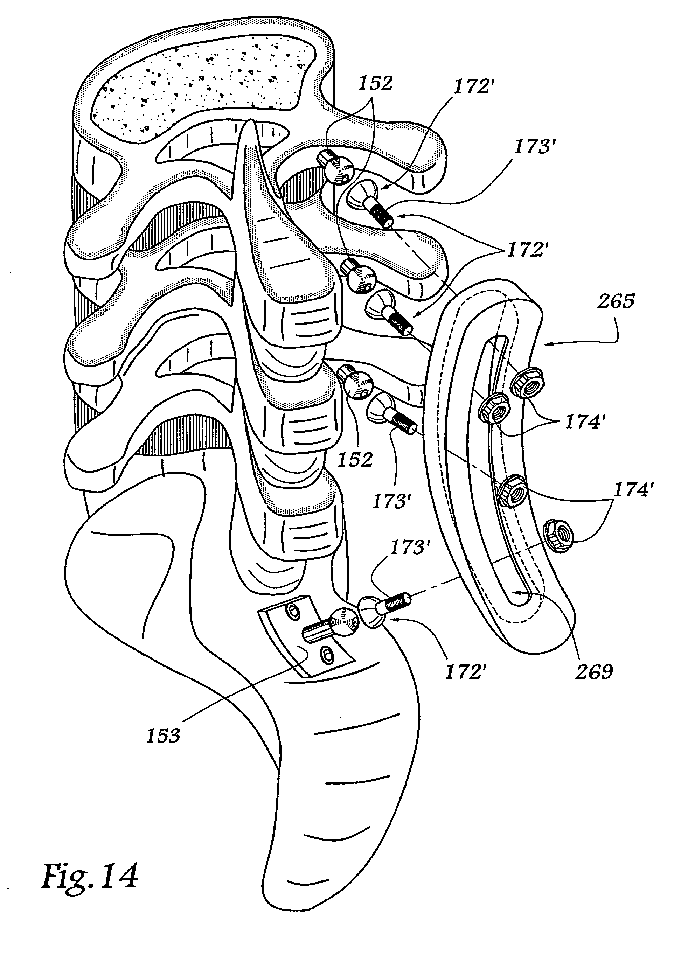

La figure 14 illustre une variante supplémentaire de l'invention. Il est prévu trois vis pédiculaires 152, une plaque sacrée 153, ainsi qu'une plaque allongée de liaison 265. La tête sphérique de chaque vis 152 ou de la plaque 153 est reçue dans le volume intérieur d'un élément intermédiaire correspondant 172'.

En outre, chaque tige filetée 173' d'un élément intermédiaire correspondant 172' est apte à pénétrer dans la lumière 269 de la plaque 265, de façon à coopérer avec un écrou correspondant 174'.

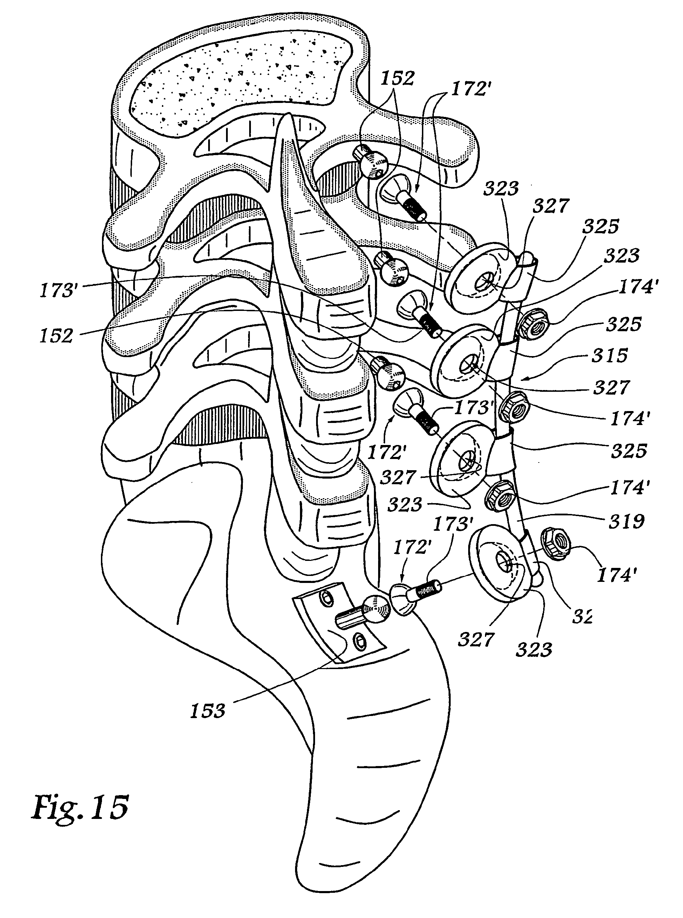

La figure 15 illustre une dernière variante de réalisation de l'invention.

Il est prévu un organe de liaison 315, comprenant une tige 319 sur laquelle sont disposés des disques 323, susceptibles d'être fixés au moyen de colliers 325.

L'orifice 327 de chaque disque 323 est apte à recevoir la tige filetée 173' d'un élément intermédiaire 172', qui coopérère avec la tête sphérique d'une vis 152, ou bien d'une plaque sacrée 153. Chaque tige 173' est par ailleurs apte à recevoir un écrou 174'.

L'invention permet de réaliser les objectifs précédemment mentionnés.

En effet, les différents éléments constitutifs du dispositif de liaison intervertébral de l'invention possèdent une structure relativement simple.

L'assemblage de ces éléments est particulièrement et peut être réalisé par un chirurgien, sans que ce dernier n'ait à mettre en oeuvre une force physique importante.

Le montage des différents éléments du dispositif de liaison de l'invention n'induit par ailleurs aucune déformation de ceux-ci, ce qui est avantageux en termes de fiabilité mécanique.

Il est à noter que la présence de l'élément intermédiaire autorise le montage mutuel de l'élément fixe et de l'élément mobile, quand bien même il n'existe pratiquement aucun débattement en rotation entre ces deux éléments.

Enfin, une fois le dispositif de liaison intervertébral de l'invention assemblé, il possède une résistance élevée à l'égard des contraintes mécaniques, s'exerçant notamment en traction. En effet, la présence de l'élément intermédiaire permet de ne transmettre que dans une très faible mesure les éventuels efforts auxquels est soumis le dispositif de liaison conforme à l'invention.

## Revendications

1. Dispositif de liaison intervertébral, destiné à relier au moins deux vertèbres entre elles, ce dispositif étant **caractérisé en ce qu'**il comprend :
- au moins un élément fixe (2 ; 2' ; 2'' ; 52, 53 ; 152, 153), solidaire d'une vertèbre ou du sacrum,
- au moins un élément mobile de liaison (10 ; 10'; 10"; 60 ; 110 ; 165 ; 215 ; 265 ; 315) apte à se déplacer par rapport au ou à chaque élément fixe,
- ainsi qu'au moins un élément intermédiaire (22 ; 22' ; 22" ; 72 ; 172, 172') permettant l'articulation du ou de chaque élément mobile par rapport au ou à chaque élément fixe,
- **en ce que** le ou chaque élément intermédiaire est reçu dans un volume intérieur (16 ; 16' ; 116 ; 167, 169; 217, 227 ; 327) dudit élément mobile (10 ; 10' ; 10" ; 110 ; 165 ; 215 ; 265 ; 315) ou dudit élément fixe (52, 53),
- **en ce que** l'élément fixe (2 ; 2'; 2" ; 152, 153) ou l'élément mobile (60) est reçu au moins partiellement dans un volume intérieur (26 ; 26') de l'élément intermédiaire,
- et **en ce qu'**il est prévu des moyens permettant la solidarisation, au moins en translation, dudit élément fixe (2; 2' ; 2" ; 152, 153) ou dudit élément mobile (60) par rapport audit élément intermédiaire, ces moyens de solidarisation en translation comprenant le pourtour du débouché (27), non déformable, du volume intérieur (26) de l'élément intermédiaire (22),
- **en ce que** le ou chaque élément intermédiaire (22 ; 22' ; 172, 172') est reçu dans un volume intérieur de l'élément mobile (10 ; 10'; 10" ; 110 ; 165 ; 215 ; 265 ; 315), **en ce que** l'élément fixe (2 ; 2' ; 2" ; 152, 153) est reçu au moins partiellement dans un volume intérieur de l'élément intermédiaire, et **en ce qu'**il, est prévu des moyens permettant la solidarisation, au moins en translation, de l'élément fixe par rapport à l'élément intermédiaire, **en ce que** ledit élément fixe (2 ; 2' ; 2") possède au moins un degré de liberté en rotation par rapport à l'élément intermédiaire (22 ; 22' ; 22"),
**en ce que** ledit élément fixe (2 ; 2' ; 2") possède une surface extérieure sphérique, apte à coopérer avec une surface intérieure (28) sphérique correspondante de l'élément intermédiaire (22 ; 22' ; 22"), et
**en ce que** la surface extérieure sphérique dudit élément fixe est pourvue d'un méplat équatorial (6; 6') d'introduction dudit élément fixe (2; 2'; 2") dans le volume intérieur de l'élément intermédiaire (22 ; 22' ; 22").

2. Dispositif selon la revendication 1, **caractérisé en ce que** ledit élément intermédiaire (22 ; 22', 22" ; 172) est solidaire en translation par rapport audit élément mobile (10 ; 10' ; 10" ; 110; 165 ; 215).

3. Dispositif selon la revendication 2, **caractérisé en ce que** le débouché (16') du volume intérieur (16) de l'élément mobile (10) possède un pourtour non déformable, apte à solidariser en translation l'élément intermédiaire par rapport à l'élément mobile.

4. Dispositif selon la revendication 2 ou 3, **caractérisé en ce que** ledit élément intermédiaire (22 ; 22' ; 22") possède une surface extérieure (24) sphérique tronquée, et coopère avec une surface intérieure (18) sphérique correspondante, dont est pourvu ledit élément mobile (10 ; 10' ; 10").

5. Dispositif selon la revendication 4, **caractérisé en ce que** l'élément fixe (2') possède une tête (4') reçue dans le volume intérieur de l'élément intermédiaire (22'), et **en ce que** ladite tête, l'élément intermédiaire et le volume intérieur dudit élément mobile (10') sont concentriques.

6. Dispositif selon la revendication 2, **caractérisé en ce que** ledit élément intermédiaire (172') est solidaire également en rotation par rapport à l'élément mobile (165 ; 215 ; 265 ; 315).

7. Dispositif selon la revendication 1, **caractérisé en ce que** l'élément intermédiaire (22) possède au moins un degré de liberté, au moins en translation, par rapport audit premier élément mobile (110).

8. Dispositif selon la revendication 7, **caractérisé en ce que** ledit élément mobile (110) possède une portion tubulaire (112), le long de laquelle est apte à coulisser ledit élément intermédiaire (22).

9. Dispositif selon l'une des revendications 2 à 8, **caractérisé en ce que** ledit élément mobile (10 ; 10' ; 10" ; 110) possède une échancrure (20 ; 120) d'introduction de l'élément intermédiaire (22 ; 22' ; 22") dans le volume intérieur (16 ; 116) de cet élément mobile.

10. Dispositif selon la revendication 9, **caractérisé en ce que** ledit élément fixe (2) est muni d'un organe de butée (32), notamment amovible, permettant de limiter le pivotement de l'élément intermédiaire (22) par rapport à l'élément fixe (2).

11. Dispositif selon l'une quelconque des revendications 1 à 9, **caractérisé en ce que**, en service, l'élément fixe (2"') est solidarisé à l'élément intermédiaire (22"'), à la fois en translation et en rotation.

12. Dispositif selon la revendication 1, **caractérisé en ce que** le ou chaque élément intermédiaire (72) est reçu dans un volume intérieur de l'élément fixe (52, 53), **en ce que** l'élément mobile (60) est reçu au moins partiellement dans un volume intérieur de l'élément intermédiaire, et **en ce qu'**il est prévu des moyens permettant la solidarisation, au moins en translation, de l'élément mobile par rapport à l'élément intermédiaire.

13. Dispositif selon l'une quelconque des revendications précédentes, **caractérisé en ce que** l'élément fixe est une vis pédiculaire (2 ; 2'; 2"; 52, 152).
pédiculaire (2 ; 2' ; 2" ; 52, 152).

14. Dispositif selon l'une quelconque des revendications précédentes, **caractérisé en ce que** l'élément fixe est une plaque (53, 153) apte à être fixée sur le sacrum.

## Claims

1. An intervertebral linking device designed to connect at least two vertebrae together, the device being **characterised in that** it comprises:
- at least one fixed member (2; 2'; 2"; 52, 53; 152, 153) fixedly attached to a vertebra or the sacrum,
- at least one movable linking member (10; 10'; 10"; 60; 110; 165; 215; 265; 315) which can move in relation to the one or more fixed members,
- together with at least one intermediate member (22; 22'; 22"; 72; 172, 172') which provides an articulation between the or all the movable members in relation to the or all fixed members,
- **in that** the or all intermediate members are received within an internal space (16; 16'; 116; 167, 169; 217, 227; 327) in the said moving member (10; 10'; 10"; 110; 165; 215; 265; 315) or the said fixed member (52, 53),
- **in that** the fixed member (2' 2'; 2"; 152, 153) or the moving member (60) are at least partly received within an internal space (26; 26') in the intermediate member,
- and **in that** means are provided to enable the said fixed member (2; 2'; 2"; 152, 153) or the said moving member (60) to be fixedly attached in relation to the said intermediate member, these means preventing lateral movement comprising the perimeter of the non-deformable opening (27) of the interior space (26) in the intermediate member (22),
- **in that** the or all the intermediate members (2; 22'; 172, 172') are received within an internal space in the moving member (10; 10'; 10"; 110; 165; 215; 265; 315), **in that** the fixed member (2; 2'; 2"; 152, 153) is at least partly received within an internal space of the intermediate member, and **in that** means are provided to prevent at least lateral movement of the fixed member in relation to the intermediate member,
**in that** the said fixed member (2; 2'; 2") has at least one degree of freedom in rotation in relation to the intermediate member (22; 22'; 22"),
**in that** the said fixed member (2; 2'; 2") has a spherical external surface capable of acting in concert with a corresponding spherical internal surface (28) of the intermediate member (22; 22'; 22"), and
**in that** the spherical external surface of the said fixed member is provided with an equatorial flat (6; 6') for insertion of the said fixed member (2; 2'; 2") within the internal space in the intermediate member (22; 22'; 22").

2. A device according to Claim 1, **characterised in that** the said intermediate member (22; 22'; 22"; 172) is fixedly attached with regard to lateral movement in relation to the said moving member (10; 10'; 10"; 110; 165; 215).

3. A device according to Claim 2, **characterised in that** the opening (16') of the internal space (16) of the moving member (10) has a non-deformable perimeter which will prevent lateral movement of the intermediate member in relation to the moving member.

4. A device according to Claim 2 or Claim 3, **characterised in that** the said intermediate member (22; 22'; 22") has a truncated spherical external surface (24) and acts in concert with a corresponding spherical internal surface (18) with which the said moving member (10; 10'; 10") is provided.

5. A device according to Claim 4, **characterised in that** the fixed member (2') has a head (4') which is received within the internal space in the intermediate member (22') and **in that** the said head, the intermediate member and the internal space in the moving member (10') are concentric.

6. A device according to Claim 2, **characterised in that** the said intermediate member (172') is also fixedly attached with regard to rotation in relation to the moving member (165; 215; 265; 315).

7. A device according to Claim 1, **characterised in that** the intermediate member (22) has at least one degree of freedom, at least laterally, in relation to the said first moving member (110).

8. A device according to Claim 7, **characterised in that** the said moving member (110) has a tubular portion (112) along which the said intermediate member (22) can slide.

9. A device according to any one of Claims 2 to 8, **characterised in that** the said moving member (10; 10'; 10"; 110) has an embayment (20; 120) for insertion of the intermediate member (22; 22'; 22") within the internal space (16; 116) in this moving member.

10. A device according to Claim 9, **characterised in that** the said fixed member (2) is provided with a stop member (32), in particular a movable one, which can limit pivoting of the intermediate member (22) in relation to the fixed member (2).

11. A device according to any one of Claims 1 to 9, **characterised in that** when in use the fixed member (2"') is fixedly attached to the intermediate member (22"') with regard to both lateral movement and rotation.

12. A device according to Claim 1, **characterised in that** each intermediate member (72) is received within an internal space in the fixed member (52, 53), **in that** the moving member (60) is at least partly received into an internal space in the intermediate member, and **in that** means are provided to enable the moving member to be fixedly attached to the intermediate member at least with regard to lateral movement.

13. A device according to any one of the preceding claims, **characterised in that** the fixed member is a pedicle screw (2; 2'; 2"; 52, 152).

14. A device according to any one of the preceding claims, **characterised in that** the fixed member is a plate (53, 153) which is designed to be attached to the sacrum.

## Patentansprüche

1. Zwischenwirbel-Verbindungsvorrichtung, die vorgesehen ist, mindestens zwei Wirbel miteinander zu verbinden, wobei die Vorrichtung **dadurch gekennzeichnet ist, dass** sie aufweist:
- mindestens ein ortsfestes Element (2; 2'; 2"; 52, 53; 152, 153), das formschlüssig mit einem Wirbel oder mit dem Kreuzbein verbunden ist,
- mindestens ein bewegbares Anschlusselement (10; 10'; 10''; 60; 110; 165; 215; 265; 315), das fähig ist, sich relativ zu dem oder zu jedem ortsfesten Element zu bewegen,
- sowie mindestens ein Zwischenelement (22; 22'; 22''; 72; 172; 172'), das das gelenkige Anschließen des oder jedes bewegbaren Elements relativ an das oder jedes ortsfeste Element ermöglicht,
- dass das oder jedes Zwischenelement in einem Innenvolumen (16; 16'; 116; 167, 169; 217, 227; 327) des bewegbaren Elements (10; 10'; 10''; 110; 165; 215; 265; 315) oder des ortsfesten Elements (52, 53) aufgenommen ist,
- dass das ortsfeste Element (2; 2'; 2''; 152, 153) oder das bewegbare Element (60) zumindest teilweise in einem Innenvolumen (26; 26') des Zwischenelements aufgenommen ist,
- und dass Mittel vorgesehen sind, die das formschlüssige Anschließen des ortsfesten Elements (2; 2'; 2''; 152, 153) oder des bewegbaren Elements (60) zumindest in Translation relativ an das Zwischenelement ermöglichen, wobei die Mittel zum formschlüssigen Anschließen in Translation den nicht verformbaren äußeren Umfang der Mündungsöffnung (27) des Innenvolumens (26) des Zwischenelements (22) aufweisen,
- dass das oder jedes Zwischenelement (22; 22'; 172, 172') in einem Innenvolumen des bewegbaren Elements (10; 10'; 10''; 110; 165; 215; 265; 315) aufgenommen ist, das ortsfeste Element (2; 2'; 2''; 152, 153) zumindest teilweise in einem Innenvolumen des Zwischenelements aufgenommen ist, und dass Mittel vorgesehen sind, die das formschlüssige Anschließen des ortsfesten Elements zumindest in Translation relativ an das Zwischenelement ermöglichen,
- dass das ortsfeste Element (2; 2'; 2'') mindestens einen Rotationsfreiheitsgrad relativ zu dem Zwischenelement (22; 22'; 22'') aufweist,
- dass das ortsfeste Element (2; 2'; 2'') eine sphärische Außenfläche aufweist, die fähig ist, mit einer korrespondierenden sphärischen Innenfläche (28) des Zwischenelements (22; 22'; 22'') zusammenzuwirken, und
- dass die sphärische Außenfläche des ortsfesten Elements mit einer äquatorialen Abflachung (6; 6') zum Einführen des ortsfesten Elements (2; 2'; 2'') in das Innenvolumen des Zwischenelements (22; 22'; 22'') ausgestattet ist.

2. Vorrichtung gemäß Anspruch 1, **dadurch gekennzeichnet, dass** das Zwischenelement (22; 22'; 22''; 172) relativ zu dem bewegbaren Element (10; 10'; 10''; 119; 165; 215) formschlüssig in Translation angeschlossen ist.

3. Vorrichtung gemäß Anspruch 2, **dadurch gekennzeichnet, dass** die Mündungsöffnung (16') des Innenvolumens (16) des bewegbaren Elements (10) einen nicht verformbaren äußeren Umfang aufweist, der geeignet ist, das Zwischenelement relativ zu dem bewegbaren Element in Translation formschlüssig anzuschließen.

4. Vorrichtung gemäß Anspruch 2 oder 3, **dadurch gekennzeichnet, dass** das Zwischenelement (22; 22'; 22'') eine abgestumpfte sphärische Außenfläche (24) aufweist und mit einer korrespondierenden sphärischen Innenfläche (18) zusammenwirkt, mit der das bewegbare Element (10; 10'; 10'') ausgestattet ist.

5. Vorrichtung gemäß Anspruch 4, **dadurch gekennzeichnet, dass** das ortsfeste Element (2') einen Kopf (4') aufweist, der in dem Innenvolumen des Zwischenelements (22') aufgenommen ist, und dass der Kopf, das Zwischenelement und das Innenvolumen des bewegbaren Elements (10') konzentrisch sind.

6. Vorrichtung gemäß Anspruch 2, **dadurch gekennzeichnet, dass** das Zwischenelement (172') relativ zu dem bewegbaren Element (165; 215; 265; 315) auch drehbar formschlüssig angeschlossen ist.

7. Vorrichtung gemäß Anspruch 1, **dadurch gekennzeichnet, dass** das Zwischenelement (22) relativ zu dem ersten bewegbaren Element (110) mindestens einen Freiheitsgrad mindestens in Translation aufweist.

8. Vorrichtung gemäß Anspruch 7, **dadurch gekennzeichnet, dass** das bewegbare Element (110) einen röhrenförmigen Abschnitt (112) aufweist, entlang dem das Zwischenelement (22) fähig ist, zu gleiten.

9. Vorrichtung gemäß einem der Ansprüche 2 bis 8, **dadurch gekennzeichnet, dass** das bewegbare Element (10; 10'; 10''; 110) eine Aussparung (20; 120) zum Einführen des Zwischenelements (22; 22'; 22'') in das Innenvolumen (16; 116) des bewegbaren Elements aufweist.

10. Vorrichtung gemäß Anspruch 9, **dadurch gekennzeichnet, dass** das ortsfeste Element (2) mit einem insbesondere herausnehmbaren Anschlagelement (32) versehen ist, das ein Beschränken der Schwenkbewegung des Zwischenelements (22) relativ zu dem ortsfesten Element (2) ermöglicht.

11. Vorrichtung gemäß einem der Ansprüche 1 bis 9, **dadurch gekennzeichnet, dass** das ortsfeste Element (2''') in Benutzung gleichzeitig sowohl in Translation als auch drehbar formschlüssig mit dem Zwischenelement (22'') verbunden ist.

12. Vorrichtung gemäß Anspruch 1, **dadurch gekennzeichnet, dass** das oder jedes Zwischenelement (72) in einem Innenvolumen des ortsfesten Elements (52, 53) aufgenommen ist, das bewegbare Element (60) zumindest teilweise in einem Innenvolumen des Zwischenelements aufgenommen ist, und Mittel vorgesehen sind, die das formschlüssige Anschließen des bewegbaren Elements zumindest in Translation relativ zu dem Zwischenelement ermöglichen.

13. Vorrichtung gemäß einem der vorhergehenden Ansprüche, **dadurch gekennzeichnet, dass** das ortsfeste Element eine Pedikelschraube (2; 2'; 2 " ; 52, 152) ist.

14. Vorrichtung gemäß einem der vorhergehenden Ansprüche, **dadurch gekennzeichnet, dass** das ortsfeste Element eine Platte (53, 153) ist, die am Kreuzbein befestigbar ist.
